# EUROPÄISCHE PATENTANMELDUNG

(11) **EP 3 685 882 A1**
(43) Veröffentlichungstag der Anmeldung: **29.07.2020**
(21) Anmeldenummer: 19153974.1
(22) Anmeldetag: 28.01.2019
(51) Int. Cl.: A61N 1/375, A61N 1/372

(54) **DURCHFÜHRUNG MIT INTEGRIERTEM HF-FENSTER FÜR EINE DATENTELEMETRIE OHNE EXTERNE ANTENNE**

(71) Anmelder: BIOTRONIK SE & Co. KG, 12359 Berlin (DE)
(72) Erfinder: STARKE, Marcel, 15732 Eichwalde (DE); ROMBERG, Jan, 12347 Berlin (DE)
(74) Vertreter: Keck, Hans-Georg

(57) **Zusammenfassung**

Die Erfindung betrifft eine implantierbare medizinisches Vorrichtung (1), mit: einer Schaltung (2), die dazu konfiguriert ist, eine elektromagnetische Welle zur Datenübertragung zu erzeugen, wobei die Schaltung eine Antenne zum Transmittieren der mittels der Schaltung (2) erzeugten elektromagnetischen Welle aufweist, einem elektrisch leitfähigen Gehäuse (4), das einen Innenraum (I) umgibt, in dem die Schaltung (2) angeordnet ist, wobei das Gehäuse (4) eine Durchgangsöffnung (5) zum Durchführen zumindest eines elektrischen Leiters (6) aufweist, der von einem elektrisch isolierenden Träger (7) umgeben ist, der die Durchgangöffnung (5) hermetisch verschließt, und einem am Gehäuse (4) festgelegten Header (8), der über der Durchgangsöffnung (5) angeordnet ist. Erfindungsgemäß ist vorgesehen, dass der Träger ein Fenster (10) aufweist, das dazu ausgebildet ist, eine mittels der Antenne (3) erzeugte elektromagnetische Welle durchzulassen, wobei die Antenne (3) und/oder die Schaltung (2) auf einer dem Innenraum (I) zugewandten Innenseite (10a) des Fensters (10) angeordnet ist und mit der Innenseite (10a) verbunden ist, oder wobei die Antenne (3) in das Fenster (10) eingebettet ist.

## Beschreibung

Die Erfindung betrifft eine implantierbare medizinische Vorrichtung.

Medizinische Implantate müssen oftmals in der Lage sein, Daten mittels einer Datentelemetrie an ein z.B. außerhalb des Körpers einen Patienten befindliches externes Gerät zu senden bzw. Daten von einem externen Gerät zu empfangen.

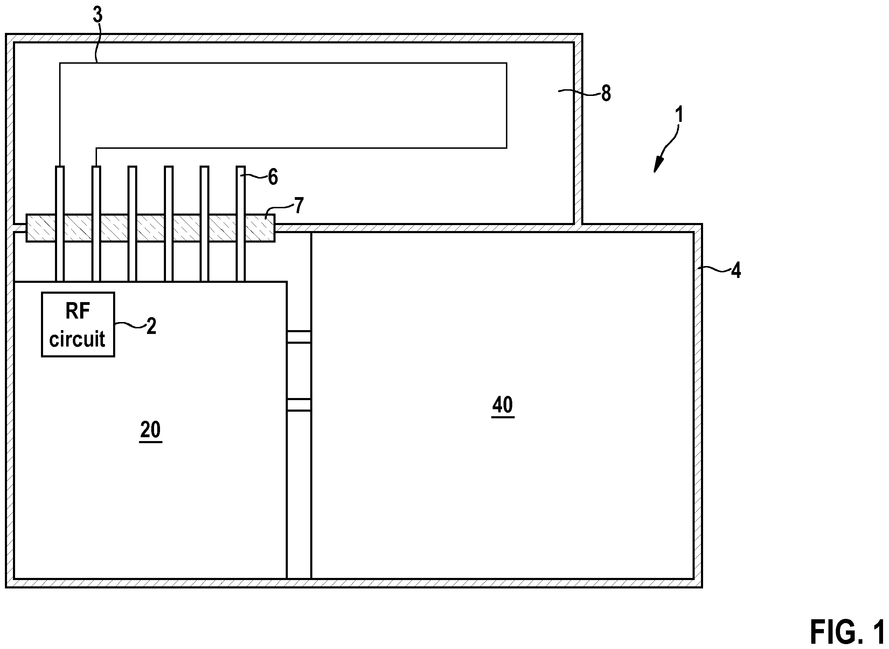

Im Stand der Technik sind diesbezüglich gemäß Figur 1 Anordnungen bekannt, bei denen sich eine HF-Schaltung 2 auf dem Implantats-Schaltkreis 20 befindet, der mittels einer im Gehäuse 4 des Implantats 1 angeordneten Batterie 40 mit Energie versorgt wird, wobei ein durch die HF-Schaltung 2 erzeugtes Signal über Stifte 6 einer elektrischen Durchführung an eine Antenne 3 übertragen wird, die im Header 8 des Implantats 1 platziert und vergossen ist. Im Folgenden wird eine solche Antenne als "externe Antenne" bezeichnet. Die Durchführung weist dabei einen Isolator 7 auf, der die Stifte 6 umgibt.

Die externe Antenne bedingt beim Stand der Technik zur Anbindung Zuleitungen und eine hermetisch dichte Durchführung. Die Ankopplung ist wegen der geometrischen Gegebenheiten von gegebenen Implantaten und Implantats-Komponenten nicht optimal. Die Antenne und die Zuleitungen verursachen zusätzliche Kosten und erhöhen die Komplexität bei der mechanischen Entwicklung (Headergestaltung, Anzahl der Durchführungsanschlüsse etc.).

Weil wegen der geometrischen Gegebenheiten die HF-Leitung und auch die Anpassung der Antenne nicht optimal ausgeführt werden kann, hat die HF-Telemetrie einen schlechten Wirkungsgrad und der Energiebedarf ist relativ hoch. Weiterhin besteht auf dem Schaltkreis besteht Flächenbedarf für die HF-Schaltung.

Aus der EP 1 923 099 A1 ist eine hermetische Kontaktdurchführung für implantierbare elektronische Geräte bekannt, wobei ein isolierender Träger in Form einer flachen Keramikscheibe eine Öffnung zur Durchführung eines Kontakts aufweist, wobei auf der Oberfläche des Trägers Areale zur Aufnahme aktiver und/oder passiver elektrischer Komponenten ausgestaltet sind, die z.B. zur Übertragung von HF-Signalen eingesetzt werden.

Weiterhin beschreibt die US 9,089,712 B2 eine implantierbare medizinische Vorrichtung, umfassend ein Gehäuse mit einem leitenden Gehäuseteil und einem Header, wobei das leitende Gehäuseteil eine Öffnung angrenzend an den Header aufweist, die mit einem dielektrischen Material verschlossen ist, wobei ein Antennensystem, das eine oder mehrere Antennen umfasst, wobei sich das Antennensystem innerhalb des leitenden Gehäuseteils unterhalb des dielektrischen Materials befindet.

Hiervon ausgehend liegt der vorliegenden Erfindung die Aufgabe zugrunde eine Antenne sowie eine zugehörige HF-Schaltung derart in die implantierbare medizinische Vorrichtung zu integrieren, dass zusätzliche aus der Durchführung herausgeführte Leiter zur Kontaktierung der Antenne vermeidbar sind. Insbesondere sollen HF-Signale möglichst effizient mittels der Antenne ausgesendet werden können. Weiterhin soll dabei vorzugsweise die Nutzung zur Verfügung stehender kommerzieller Bauteile für die Datentelemetrie ermöglicht werden.

Diese Aufgabe wird durch eine implantierbare medizinische Vorrichtung mit den Merkmalen des Anspruchs 1 gelöst.

Vorteilhafte Ausgestaltungen der Erfindung sind in den Unteransprüchen angegeben und werden nachfolgend beschrieben.

Gemäß Anspruch 1 wird eine implantierbare medizinische Vorrichtung offenbart, mit:
- einer Schaltung, die dazu konfiguriert ist eine elektromagnetische Welle zur Datenübertragung zu erzeugen, wobei die Schaltung eine Antenne zum Transmittieren der mittels der Schaltung erzeugten elektromagnetischen Welle umfasst,
- einem elektrisch leitfähigen Gehäuse, das einen Innenraum aufweist, in dem die Schaltung angeordnet ist, wobei das Gehäuse eine Durchgangsöffnung zum Durchführen zumindest eines elektrischen Leiters (z.B. in Form eines Stifts) aufweist, der von einem elektrisch isolierenden Träger umgeben ist, der die Durchgangöffhung hermetisch verschließt, und
- einem am Gehäuse festgelegten Header, der über der Durchgangsöffnung angeordnet ist, und

Erfindungsgemäß ist vorgesehen, dass der Träger ein Fenster aufweist, das für mittels der Antenne abgestrahlte elektromagnetische Wellen durchlässig ist, wobei
- die Antenne und/oder die Schaltung auf einer dem Innenraum zugewandten Innenseite des Fensters angeordnet ist bzw. sind und mit der Innenseite verbunden ist bzw. sind, oder wobei
- die Antenne in das Fenster eingebettet ist.

Der Header wird auch als Verbindungskopf bezeichnet und ist insbesondere zum Verbinden der medizinischen Vorrichtung mit einer oder mehreren weiteren Komponenten ausgebildet. Gemäß einer Ausführungsform kann es sich bei der medizinischen Vorrichtung z.B. um einen implantierbaren Herzschrittmacher handeln. In diesem Fall kann z.B. der Verbindungskopf dazu ausgebildet sein mit zumindest einer Elektrodenleitung verbunden zu werden.

Die Durchführung bzw. Vorrichtung kann mehrere elektrisch leitfähige Leiter (z.B. Stifte) aufweisen, die vom Träger umgeben sind und über die Durchgangsöffnung des Gehäuses aus einem Innenraum des Gehäuses herausgeführt sind bzw. in einen Innenraum des Headers geführt sind.

Die besagte Schaltung ist vorzugsweise dazu ausgebildet, hochfrequente (HF) elektromagnetische Wellen zu erzeugen, insbesondere im Frequenzbereich von 100 Kilohertz bis 300 Gigahertz. Die besagte Schaltung bildet insbesondere eine Telemetrie, die dazu konfiguriert, ist Daten mittels hochfrequenter elektromagnetischer Wellen zu übertragen (kurz HF-Telemetrie). Die Datenübertragung kann z.B. nach einem Bluetooth-Standard erfolgen.

Durch eine Anordnung der Komponenten für die besagte Schaltung bzw. HF-Telemetrie nahe an der Außenhaut bzw. des Gehäuses der Vorrichtung benachbart zu dem besagten Fenster und einer optionalen Integration der Antenne in die Schaltung (z.B. integrierter Sendechip), kann auf eine externe Antenne im Header mit Vorteil verzichtet werden.

Das ermöglicht die Herstellung von implantierbaren medizinischen Vorrichtungen mit HF-Telemetrie (z.B. für Bluetooth) ohne externe Antenne bei potentieller Verbesserung der Übertragungsreichweite und bei Vermeidung geräteinterner Störungen durch HF-Signale.

Gemäß einer Ausführungsform der Erfindung ist vorgesehen, dass das Fenster durch eines der folgenden Materialien gebildet ist oder eines der folgenden Materialien aufweist: ein dielektrisches Material, wie z.B. Keramik, Kunststoff, kristalline, nicht leitende Materialien wir z.B. Quarz oder amorphe nicht leitende Materialien wie z.B. Glas

Weiterhin ist gemäß einer Ausführungsform der Erfindung vorgesehen, dass die Antenne und/oder die Schaltung mit dem Fenster bzw. der Innenseite des Fensters verbunden, z.B. gelötet, gebondet oder verklebt ist.

Weiterhin ist gemäß einer Ausführungsform der Erfindung vorgesehen, dass durch das Fenster bzw. ein entsprechendes Material keine elektrischen Leiter hindurchgeführt sind.

Weiterhin ist gemäß einer Ausführungsform der Erfindung vorgesehen, dass die Schaltung als integrierte Halbleiterschaltung ausgebildet ist.

Weiterhin ist gemäß einer Ausführungsform der Erfindung vorgesehen, dass die Antenne zusammen mit der Schaltung als integrierte Halbleiterschaltung ausgebildet ist (also insbesondere nicht als diskretes Bauteil), wobei insbesondere die integrierte Halbleiterschaltung auf der dem Innenraum zugewandten Innenseite des Fensters angeordnet ist und dabei mit der Innenseite des Fensters verbunden ist (z.B. verlötet, gebondet oder verklebt ist)

Weiterhin ist gemäß einer Ausführungsform der Erfindung vorgesehen, dass die implantierbare medizinische Vorrichtung eine im Träger angeordnete metallische Schicht zur Abschirmung elektromagnetischer Felder aufweist, wobei die metallische Schicht eine Durchgangsöffnung aufweist, durch die sich das Fenster bzw. ein entsprechendes Material hindurch erstreckt.

Gemäß einer Ausführungsform der Erfindung ist vorgesehen, dass der Träger aus mehreren Lagen gebildet ist.

Weiterhin kann hinsichtlich des Trägers gemäß einer Ausführungsform der Erfindung vorgesehen sein, dass der Träger bzw. die einzelnen Lagen ein keramisches Material wie z.B. Al2O3 oder LTCC-Keramik-Gemische wie L8 LTCC von Ferro aufweisen oder aus einem keramischen Material bestehen.

Gemäß einer Ausführungsform ist die metallische Schicht in den Träger eingebettet. Weiterhin ist gemäß einer Ausführungsform vorgesehen, dass sich die metallische Schicht im Innenraum des Gehäuses entlang der gesamten Fläche der Durchgangsöffnung vor der Durchgangsöffnung erstreckt. Hierbei ist die metallische Schicht durch das Fenster bzw. einen entsprechenden Materialbereich und die Durchgangsöffnungen für die Leiter bzw. Stifte unterbrochen.

Weiterhin ist gemäß einer Ausführungsform der Erfindung vorgesehen, dass die metallische Schicht durch eine Metallisierung einer Lage des Trägers gebildet ist.

Gemäß einer weiteren Ausführungsform der Erfindung ist vorgesehen, dass über der Schaltung und der Antenne ein metallischer Deckel angeordnet ist, um einen angrenzenden Bereich des Innenraums des Gehäuses gegen elektromagnetische Felder bzw. HF-Signale der besagten Schaltung abzuschirmen.

Weiterhin ist gemäß einer Ausführungsform der Erfindung vorgesehen, dass der metallische Deckel mit der metallischen Schicht verbunden ist. Der Deckel und die metallische Schicht umgeben dabei gemeinsam die Schaltung und die Antenne. In einer weiteren Ausführungsform kann der Deckel oder die metallische Schicht auf einem zur Schirmung geeigneten elektrischen Potential angebunden werden.

Weiterhin ist vorzugsweise gemäß einer Ausführungsform vorgesehen, dass die metallische Schicht eine Durchgangsöffnung aufweist, durch die der mindestens eine Leiter bzw. Stift geführt ist.

Gemäß einer Ausführungsform kann die Antenne, insbesondere für den Fall, dass sie als diskretes Bauteil ausgeführt ist, als ein SMD-Bauelement ausgebildet sein. Derartige oberflächenmontierte Bauelemente weisen im Gegensatz zu Bauelementen, die mittels Durchsteckmontage montiert werden, keine Drahtanschlüsse auf, sondern werden mittels lötfähiger Anschlussflächen direkt auf eine Leiterplatte bzw. einen Träger gelötet.

Gemäß dem alternativen Aspekt der Erfindung kann die Antenne in den das Fenster vollständig eingebettet sein. Hierbei kann die Antenne über eine Durchkontaktierung mit der Schaltung verbunden sein. Diese kann z.B. auf der Innenseite des Fensters angeordnet sein und kann wiederum mit dieser Innenseite verbunden sein (z.B. durch Verkleben).

Aufgrund der Erfindung kann die Sendereichweite mit Vorteil gesteigert werden. Weiterhin erhöht sich durch die Einsparung von Energie für die HF-Telemetrie die Lebensdauer der Vorrichtung. Ferner kann auf eine im Header angeordnete Antenne verzichtet werden, so dass der Header kompakter gestaltet werden kann. Insbesondere sind zusätzliche Stifte zur Kontaktierung einer im Header angeordneten Antenne vermeidbar.

Gemäß weiterer bevorzugter Ausführungsformen der vorliegenden Erfindung handelt es sich bei der implantierbaren medizinischen Vorrichtung um eines oder folgenden Geräte oder um eine Kombination aus den folgenden Geräten:
- kardiales Therapie-, Monitoring- oder Diagnosegerät, wie z.B. ein kardialer Loop-Recorder, ein Herzschrittmacher, ein implantierbarer Kardioverter-Defibrillator, ein kardiales Resynchronisationsgerät (CRT-Gerät),
- ein Therapie-, Monitoring- oder Diagnosegerät für das Nervensystem, wie z.B. ein Rückenmarkstimulator, ein Vagusnerv-Stimulator, ein Hirnstimulator,
- ein sonstiges Therapie-, Monitoring- oder Diagnosegerät, das mit einer Drahtlostelemetriefunktion ausgestattet ist.

Weitere Merkmale und Vorteile der Erfindung sollen bei der Figurenbeschreibung von Ausführungsbeispielen der Erfindung anhand der Figuren erläutert werden. Es zeigen:
- Fig. 1: eine implantierbare medizinische Vorrichtung mit einer im Header angeordneten Antenne nach dem Stand der Technik;
- Fig. 2: eine schematische Schnittansicht einer Ausführungsform einer erfindungsgemäßen implantierbaren medizinischen Vorrichtung;
- Fig. 3: eine schematische Schnittansicht einer weiteren Ausführungsform einer erfindungsgemäßen implantierbaren medizinischen Vorrichtung;
- Fig. 4: eine schematische Schnittansicht (A) eines Trägers einer Ausführungsform einer erfindungsgemäßen Vorrichtung mit einer in den Träger eingebetteten Antenne, sowie eine Draufsicht (B) auf die Antenne entlang der Richtung A; und
- Fig. 5: die Abstrahlungen von HF-Signalen bei einer erfindungsgemäßen Vorrichtung.

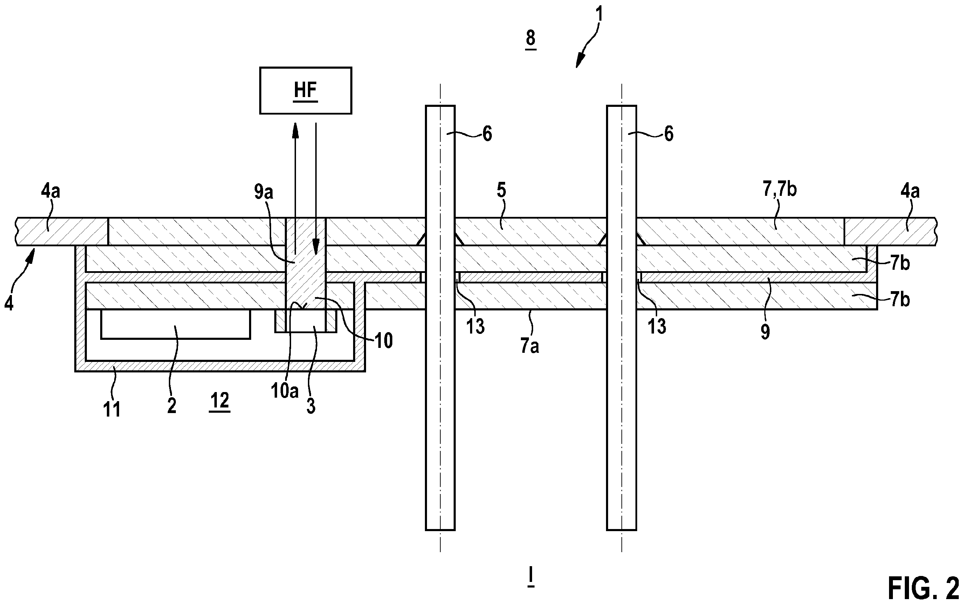

Die Figur 2 zeigt eine Ausführungsform einer erfindungsgemäßen implantierbaren medizinischen Vorrichtung 1. Die Vorrichtung, bei der es sich z.B. um einen Herzschrittmacher handeln kann, weist eine Schaltung 2, die dazu konfiguriert ist, eine elektromagnetische Welle zur Datenübertragung zu erzeugen, insbesondere in Form von HF-Signalen. Die Vorrichtung 1 weist weiterhin eine mit der Schaltung 2 verbundene Antenne 3 auf, die zum Transmittieren der mittels der Schaltung 2 erzeugten elektromagnetischen Wellen ausgebildet ist. Die Schaltung 2 ist in einem Innenraum I eines elektrisch leitfähigen Gehäuses 4 der Vorrichtung 1 angeordnet, wobei das Gehäuse 4 eine Durchgangsöffnung 5 zum Durchführen zumindest eines elektrischen Leiters, hier mehrere Stifte 6, aufweist, die von einem elektrisch isolierenden Träger 7 umgeben sind und dabei jeweils mit ihren Enden aus dem Träger 7 herausstehen. Die Stifte 6 werden so vom Innenraum I des Gehäuses 4 in einen Header bzw. Verbindungskopf 8 geführt, der über der Durchgangsöffnung 5 angeordnet ist und mit dem Gehäuse 4 verbunden ist. Der Träger 7 verschließt des Weiteren die Durchgangsöffnung 5 hermetisch. Zum Abschirmen von elektromagnetischen Feldern bzw. Interferenzen (EMI) kann in den Träger 7 eine metallische Schicht 9 eingebettet sein, die z.B. als Metallisierung einer Lage 7b des (mehrlagigen) Trägers 7 ausgebildet sein kann. Die einzelnen Lagen 7b des Trägers 7 bzw. Durchführungsisolators können aus einem keramischen Material bestehen.

Zum Durchlassen der durch die Schaltung 2 erzeugten Signale bzw. elektromagnetischen Wellen (HF) ist vorgesehen, dass die Durchführung bzw. der Träger 7 ein Fenster 10 aufweist, das z.B. durch einen sich durch den Träger 7 erstreckenden Bereich des Trägers 7 gebildet ist, der vorzugsweise aus einem Material besteht, das durchlässig für die mittels der Antenne 3 abgestrahlten Signale bzw. elektromagnetischen Wellen ist. Bei dem Material des Fensters 10 kann es sich z.B. um wie z.B. Keramik, Kunststoff, kristalline, nicht leitende Materialien wir z.B. Quarz oder amorphe nicht leitende Materialien wie z.B. Glas handeln.

Gemäß Figur 2 können die Schaltung 2 und die damit verbundene Antenne 3 nebeneinander auf einer dem Innenraum I zugewandten Seite 7a des Trägers 7 benachbart zum Fenster 10 angeordnet sein, wobei vorzugsweise die Antenne 3 auf einer dem Innenraum I zugewandten Innenseite 10a des Fenster 10 angeordnet ist und mit dieser Innenseite 10a verbunden ist. Die Antenne 3 kann somit elektromagnetische Wellen direkt durch das Fenster 10 hindurch aus dem Gehäuse 4 heraus abstrahlen. Bei der Antenne 3 kann es sich um ein SMD-Bauteil handeln. Die Schaltung 2 kann separat dazu als integrierte Halbleiterschaltung 2 ausgeführt sein.

Weiterhin ist vorgesehen, dass über der Schaltung 2 und der Antenne 3 ein metallischer Deckel 11 angeordnet ist, der einen angrenzenden Bereich 12 des Innenraums I des Gehäuses 4 gegen elektromagnetische Felder abschirmt. Dabei ist der metallische Deckel 11 mit der metallischen Schicht 9 verbunden, so dass der Deckel 11 und die Schicht 9 die Schaltung 2 und die Antenne 3 umgeben. Die metallische Schicht 9 erstreckt sich vorzugsweise entlang des Trägers 7 auf gesamter Länge der Durchgangsöffnung 5 und weist daher vorzugsweise zur Durchführung der Stifte 6 entsprechende Durchgangsöffnungen 13 auf.

Der Träger 7 bzw. Durchführungsisolator 7 kann über einen Flansch 4a des Gehäuses 4 am Gehäuse 4 festgelegt sein. Der Flansch 4a kann bspw. mittels einer Schweißverbindung mit einem angrenzenden Bereich des Gehäuses 4 verbunden sein. Der Flansch 4a kann aus Titan gefertigt sein.

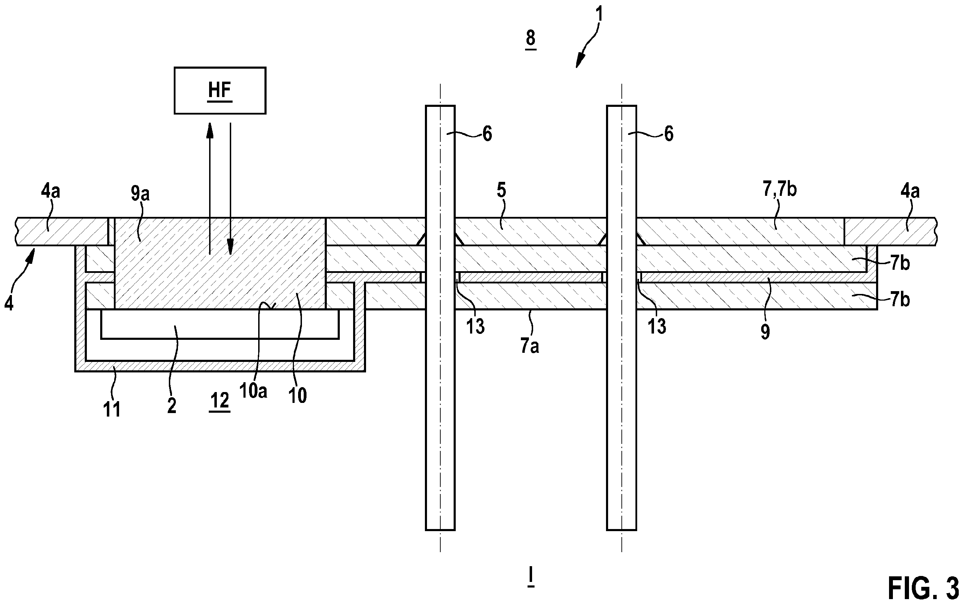

Die Figur 3 zeigt eine Abwandlung der in der Figur 2 gezeigten Ausführungsform, wobei hier im Unterschied zur Figur 2 die Antenne 3 zusammen mit der Schaltung 2 als integrierte Halbleiterschaltung ausgebildet ist. Hierbei ist nun die integrierte Schaltung 2 auf der dem Innenraum I zugewandten Innenseite 10a des Fensters 10 angeordnet.

In den Ausführungsformen gemäß Figuren 2 und 3 bildet also das Fenster 10 bzw. der Träger 7 jeweils ein Schaltungssubstrat zum Tragen der Schaltung 2 bzw. Antenne 3.

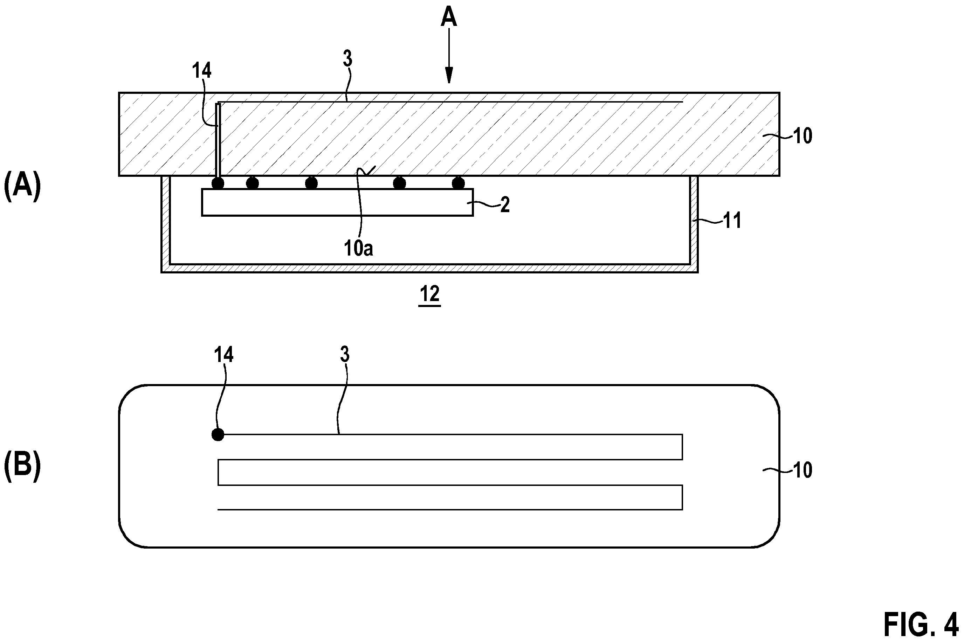

Die Figur 4 zeigt eine weitere Möglichkeit zur Anordnung der Antenne 3. Hier ist die Schaltung 2 wiederum als z.B. integrierte Halbleiterschaltung 2 auf der Innenseite 10a des Fensters oder der Innenseite 7a des Trägers 7 angeordnet, die dem Innenraum I des Gehäuses 4 zugewandt ist. Die Antenne 3 ist in Form eines z.B. mäanderförmigen Leiters (vgl. Fig. 4 (B)) in das Fenster 10 eingebettet und vorzugsweise über eine Durchkontaktierung 14 mit der Schaltung 2 verbunden.

Auch hier kann wiederum über der Schaltung 2 ein metallischer Deckel 11 angeordnet sein, der einen angrenzenden Bereich 12 des Innenraums I des Gehäuses 4 gegen elektromagnetische Felder abschirmt.

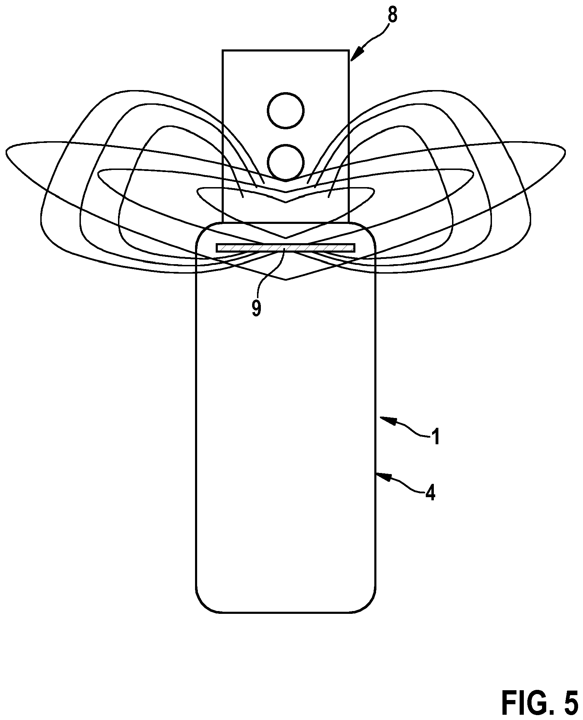

Durch die erfindungsgemäße Anordnung der Schaltung 2 bzw. Antenne 3 am bzw. im Fensters 10 bzw. nahe an der Außenhaut/Gehäuse 4 der Vorrichtung 1 kann auf eine externe Antenne im Header 8 verzichtet werden (vgl. Fig. 1). Das ermöglicht die Herstellung von implantierbaren medizinischen Vorrichtungen 1 mit HF-Telemetrie (z.B. für Bluetooth) bei potentieller Verbesserung der Übertragungsreichweite und bei Vermeidung geräteinterner Störungen durch HF-Signale aufgrund der metallischen Schicht 9 (vgl. Fig. 5). Weiterhin können zusätzliche Pins 6 der Durchführung zur Kontaktierung einer headerseitigen Antenne vermieden werden.

## Patentansprüche

1. Implantierbare medizinische Vorrichtung (1), mit:
- einer Schaltung (2), die dazu konfiguriert ist, eine elektromagnetische Welle zur Datenübertragung zu erzeugen, wobei die Schaltung eine Antenne zum Transmittieren der mittels der Schaltung (2) erzeugten elektromagnetischen Welle aufweist,
- einem elektrisch leitfähigen Gehäuse (4), das einen Innenraum (I) umgibt, in dem die Schaltung (2) angeordnet ist, wobei das Gehäuse (4) eine Durchgangsöffnung (5) zum Durchführen zumindest eines elektrischen Leiters (6) aufweist, der von einem elektrisch isolierenden Träger (7) umgeben ist, der die Durchgangöffhung (5) hermetisch verschließt, und
- einem am Gehäuse (4) festgelegten Header (8), der über der Durchgangsöffnung (5) angeordnet ist,
**dadurch gekennzeichnet,**
**dass** der Träger ein Fenster (10) aufweist, das dazu ausgebildet ist, eine mittels der Antenne erzeugte elektromagnetische Welle durch den Träger hindurch zu lassen, wobei die Antenne (3) und/oder die Schaltung (2) auf einer dem Innenraum (I) zugewandten Innenseite (10a) des Fensters (10) angeordnet ist und mit der Innenseite (10a) verbunden ist, oder wobei die Antenne (3) in das Fenster (10) eingebettet ist.

2. Implantierbare medizinische Vorrichtung nach einem der vorhergehenden Ansprüche, **dadurch gekennzeichnet, dass** das Fenster (10) aus einem der folgenden Materialien besteht oder eines der folgenden Materialien aufweist: ein dielektrisches Material, ein kristallines Material, ein nichtleitendes Material, ein amorphes nichtleitendes Material.

3. Implantierbare medizinische Vorrichtung (1) nach Anspruch 1 oder 2, **dadurch gekennzeichnet, dass** die Antenne (3) und/oder die Schaltung (2) mit der Innenseite (10a) des Fensters (10) verklebt oder gebondet oder gelötet ist.

4. Implantierbare medizinische Vorrichtung nach einem der vorhergehenden Ansprüche, **dadurch gekennzeichnet, dass** durch das Fenster (10) keine elektrischen Leiter hindurchgeführt sind.

5. Implantierbare medizinische Vorrichtung nach einem der vorhergehenden Ansprüche, **dadurch gekennzeichnet, dass** die Schaltung (2) als integrierte Halbleiterschaltung ausgebildet ist oder die Antenne (3) zusammen mit der Schaltung (2) als integrierte Halbleiterschaltung ausgebildet ist.

6. Implantierbare medizinische Vorrichtung nach einem der vorhergehenden Ansprüche, **dadurch gekennzeichnet, dass** die implantierbare medizinische Vorrichtung eine im Träger (7) angeordnete metallische Schicht (9) zur Abschirmung elektromagnetischer Felder aufweist, wobei die metallische Schicht (9) eine Durchgangsöffnung (9a) aufweist, durch die sich das Fenster (10) hindurch erstreckt.

7. Implantierbare medizinische Vorrichtung nach einem der vorhergehenden Ansprüche, **dadurch gekennzeichnet, dass** der Träger (7) aus mehreren Lagen (7b) gebildet ist.

8. Implantierbare medizinische Vorrichtung nach den Ansprüchen 6 oder 7, **dadurch gekennzeichnet, dass** die metallische Schicht (9) durch eine Metallisierung einer Lage (7b) des Trägers (7) gebildet ist.

9. Implantierbare medizinische Vorrichtung nach einem der vorhergehenden Ansprüche, **dadurch gekennzeichnet, dass** über der Schaltung (2) und der Antenne (3) ein metallischer Deckel (11) angeordnet ist, um einen angrenzenden Bereich (12) des Innenraums (I) des Gehäuses (4) gegen elektromagnetische Felder abzuschirmen.

10. Implantierbare medizinische Vorrichtung nach Anspruch 9, **dadurch gekennzeichnet, dass** der metallische Deckel (11) mit der metallischen Schicht (9) verbunden ist.

11. Implantierbare medizinische Vorrichtung nach den Ansprüchen 9 oder 10, **dadurch gekennzeichnet, dass** der Deckel oder die metallische Schicht mit einem elektrischen Potential verbunden ist, das zur elektromagnetischen Schirmung geeignet ist.

12. Implantierbare medizinische Vorrichtung nach einem der vorhergehenden Ansprüche, **dadurch gekennzeichnet, dass** die metallische Schicht (9) eine Durchgangsöffnung (13) aufweist, durch die der mindestens eine Leiter (6) geführt ist.

13. Implantierbare medizinische Vorrichtung nach einem der vorhergehenden Ansprüche, **dadurch gekennzeichnet, dass** die Antenne (3) als ein SMD-Bauelement ausgebildet ist.

14. Implantierbare medizinische Vorrichtung nach einem der Ansprüche 1 bis 2, **dadurch gekennzeichnet, dass** die in das Fenster (10) eingebettete Antenne (3) über eine Durchkontaktierung (14) mit der Schaltung (2) verbunden ist.

15. Implantierbare medizinische Vorrichtung nach mindestens einem der vorhergehenden Ansprüche, **dadurch gekennzeichnet, dass** die implantierbare medizinische Vorrichtung als eines oder folgenden Geräte oder als eine Kombination aus den folgenden Geräten ausgebildet ist:
- kardiales Monitoring-Gerät,
- Herzschrittmacher,
- implantierbarer Kardioverter-Defibrillator,
- kardiales Resynchronisationsgerät (CRT-Gerät),
- Rückenmarkstimulator,
- Vagusnerv-Stimulator,
- Hirnstimulator.
